(19) Europäisches Patentamt
European Patent Office
Office européen des brevets

(11) Veröffentlichungsnummer: **0 036 550**
**B2**

(12) # NEUE EUROPÄISCHE PATENTSCHRIFT

(45) Veröffentlichungstag der neuen Patentschrift: **16.03.88**

(51) Int. Cl.⁴: **C 07 C 43/11**, C 07 C 41/06, C 11 D 1/722

(21) Anmeldenummer: **81101739.1**

(22) Anmeldetag: **10.03.81**

(54) **Verwendung von alkoxylierten Fettalkoholen, die mit Propylenendgruppen verschlossen sind, als schaumarme säure- und alkalistabile Tenside.**

(30) Priorität: **22.03.80 DE 3011237**

(43) Veröffentlichungstag der Anmeldung: **30.09.81 Patentblatt 81/39**

(45) Bekanntmachung des Hinweises auf die Patenterteilung: **09.02.83 Patentblatt 83/6**

(45) Bekanntmachung des Hinweises auf die Entscheidung über den Einspruch: **16.03.88 Patentblatt 88/11**

(84) Benannte Vertragsstaaten: **BE CH DE FR GB IT LI SE**

(56) Entgegenhaltungen:
**DE-A-2 028 423**
**DE-A-2 049 238**
**DE-A-2 263 980**
**DE-A-2 450 667**
**DE-A-2 556 499**
**DE-A-2 556 527**
**DE-A-2 556 544**
**DE-A-2 801 793**
**DE-B-1 224 294**
**DE-B-1 520 647**
**DE-B-2 544 569**
**DE-B-2 559 654**
**DE-C-868 147**
**US-A-3 362 133**
**US-A-3 591 641**

**J. Falbe, U. Hassendt: Katalysatoren, Tenside und Mineralöladditive, Stuttgart (Thieme) 1970, S. 149**

(73) Patentinhaber: **BASF Aktiengesellschaft, Carl-Bosch- Strasse 38, D-6700 Ludwigshafen (DE)**

(72) Erfinder: **Vodrazka, Wolfgang, Dr., Alzeyer Strasse 8, D-6713 Freinsheim (DE)**
Erfinder: **Klahr, Erhard, Dr., Londoner Ring 11, D-6700 Ludwigshafen (DE)**
Erfinder: **Oppenlaender, Knut, Dr., Otto- Dill-Strasse 23, D-6700 Ludwigshafen (DE)**
Erfinder: **Trieselt, Wolfgang, Dr., Alwin- Mittasch-Platz 1, D-6700 Ludwigshafen (DE)**
Erfinder: **Stoeckigt, Dieter, Koenigstrasse 4, D-6700 Ludwigshafen (DE)**
Erfinder: **Neumann, Werner, Kantstrasse 3, D-6841 Reilingen (DE)**

**EP 0 036 550 B2**

LIBER, STOCKHOLM 1988

**Beschreibung**

Verwendung von alkoxylierten Fettalkoholen die mit Propylen endgruppenverschlossen sind als schaumarme säure- und alkalistabile Tenside.

Die Erfindung betrifft die Verwendung von alkoxylierten Fettalkoholen, deren Endgruppen mit Propylen verschlossen sind. Sie betrifft die Verwendung als Tenside, die sich durch Schaumarmut, Alkali- und vor allem auch Säurestabilität auszeichnen.

Die Reaktion von u. a. Propylen mit Polyäthylenglykolen, also reinen Ethylenoxid-Polymerisaten oder mit deren Monomethylethern in Gegenwart von sauren Ionenaustauschern ist aus der DE-AS 2 801 793 bzw. der DE-AS 2 544 569 bekannt. Man kommt hierbei zu endgruppenverschlossenen Polyethylenglykolen bzw. deren Methylethern.

Auch aus der DE-A-2 450 667 ist ein solches Verfahren bekannt, indem man wiederum Polyglycole mit Olefinen, laut Anspruch 3 mit Hexen als längskettigem Olefin, umsetzt. Endgruppenverschlossene Alkoxylate langkettiger Alkohole erhält man gemäß diesen Publikationen nicht. Aus der DE-AS 1 520 647 sind endgruppenverschlossene Ethoxylate höherer Alkohole bekannt, bei denen der Endgruppenverschluß mit Isobutylen oder höheren tertiären Olefinen durchgeführt worden ist. Ausdrücklich heißt es in dieser Publikation (siehe Spalte 5, Zeilen 38 ff. a.a.O.), daß die Umsetzung von höheren Alkoholethoxylaten mit sekundären Olefinen, wie Propylen, nicht oder nur in sehr geringem Umfang möglich sei und daß eine Bildung von entsprechenden sec-Alkylethern analytisch nicht nachweisbar sei.

Die in der DE-AS 1 520 647 beschriebenen tert. alkylendgruppenverschlossenen Alkoholethoxylate sind laut Angaben dieser Publikation alkalistabil und schaumarm; sie sind aber nicht stabil gegen Säuren, da in saurem Medium eine Etherspaltung eintritt.

Das Ziel der Erfindung bestand in der Auffindung endgruppenverschlossener Tenside, die neben den vorgenannten Eigenschaften auch die der Säurestabilität besitzen, was beispielsweise ihre Anwendung in sauren Flotten in technischen Reinigern ermöglicht.

Dieses Ziel wurde durch die Verwendung von verbindungen der Formel I erreicht, wie sie gemäß den Patentansprüchen 1 oder 2 definiert sind.

Angesichts der Tatsache, daß die entsprechenden tert.-Alkyl-polyalkylenglykolether nicht säurestabil sind, stellen die neuen sec. Alkylether einen überraschenden Fortschritt auf dem Gebiet der nichtionischen Tenside dieses Aufbauprinzips dar. Außerdem ist es überraschend, daß die Umsetzung der höheren Alkoholalkoxylate mit Propylen, wie dies bislang nur mit reinen Polyäthylenglycolen oder allenfalls deren Monomethylethern realisiert wurde, überhaupt möglich ist, nachdem gemäß DE-AS 1 520 647 diese Möglichkeit eindeutig ausgeschlossen wurde.

Ausgangsverbindungen zur Herstellung der Produkte der Formel I sind höhere Alkohole mit 8 bis 20 C-Atomen oder deren Gemische, die mit 4 bis 30 Alkylenoxidgruppen, wie sie in den Patentansprüchen definiert sind, alkoxyliert sind.

Basisalkohole sind in der Regel gesättigte, möglichst wenig Verzweigungen aufweisende Alkohole, und zwar sowohl native als auch synthetische, sowie deren Mischungen. Zu nennen seien beispielsweise Octanol, Nonanol, Decanol, Dodecanol, Tetradecanol, Hexadecanol, Octadecanol (Stearylalkohol), deren Gemische, sodann synthetische Alkoholgemische aus der Oxo- und Zieglersynthese, wie $C_9/C_{11}/$-$C_{13}/C_{15}$-Oxoalkohole, $C_8/C_{10}/$-$C_{12}/C_{14}$- und $C_{16}/C_{20}$-Ziegler-Alkohole.

Diese Alkohole sind mit 4 bis 30, vorzugsweise 5 bis 15 Ethylen-, 1,2-Propylen- und/oder 1,2-Butylenoxidgruppen, mit der Maßgabe alkoxyliert, daß der Polyalkylenoxid-Block definitionsgemäß aus 100 bis 60 Mol-% Ethylenoxidgruppen und 0 bis 40 Mol-% Propylen- und/oder Butylenoxidgruppen, vorzugsweise Propylenoxidgruppen besteht.

Bevorzugt enthält der Polyalkylenoxid-Block aber ausschließlich Ethylenoxidgruppen. Falls Propylen- und/oder Butylenoxidgruppen mitenthalten sind, können die Polyalkylenoxid-Blöcke die einzelnen Alkylenoxidgruppen statistisch verteilt enthalten (Herstellung durch Mischbegasung) oder aber in Form von individuellen Blöcken (Herstellung durch stufenweise Alkoxylierung mit den einzelnen Alkylenoxiden). Die Operationen der Alkoxylierung sind bekannt und bedürfen keiner speziellen Erläuterung.

Die weitere Umsetzung der erhaltenen Alkoxylate mit Propylen entspricht prinzipiell den Methode, wie sie aus den obengenannten DE-AS 2 544 569 und 2 801 793 bekannt sind.

Hierbei setzt man den alkoxylierten Alkohol oder ein Gemisch solcher Alkoxylate bei Temperaturen von 50 bis 170, vorzugsweise 100 bis 150°C bei 1 bis 100 bar mit Propylen in äquimolarem Verhältnis oder - vorzugsweise - mit einem Überschuß an Propylen in einem Druckgefäß (diskontinuierlich) oder einem Druckrohrreaktor (kontinuierlich) in Gegenwart eines sauren Katalysators um.

Als Katalysatoren eignen sich insbesondere stark saure Ionenaustauscher. Als derartige stark saure Ionenaustauscher kommen insbesondere Sulfogruppen tragende vernetzte Styrol/Divinylbenzol-Copolymerisate in Betracht, wie sie z. B. unter den warenzeichenrechtlich geschützten Handelsbezeichnungen ®LEWATIT SPC 118 H, LEWATIT SPC 108 H, ®AMBERLITE 200 und ®AMBERLYST 15 bekannt sind.

Die Katalysatoren sind, bezogen auf Alkoholalkoxylat zu ca. 1 bis 30 Gew.-%, vorzugsweise 5 bis 20 Gew.-%, im Reaktionsgemisch anwesend.

Die Reaktion kann ohne Lösungsmittel unter autogenem Propylendruck oder in Gegenwart von inerten Lösungsmitteln, d. h. Lösungsmitteln, die unter den Reaktionsbedingungen in das chemische Geschehen nicht eingreifen, stattfinden. Solche Lösungsmittel, die zur Suspendierung des Katalysators u. U. zweckmäßig sein

können, besitzen i. a. Siedepunkte von 35 bis 200°C, und es seien z B. Diethylether, Cyclohexan oder Tetrahydrofuran genannt.

Die Drücke, bei denen die Reaktion stattfindet, richten sich nach den gewählten Reaktionstemperaturen und der gewünschten Reaktionsdauer, die eine bis 30 Stunden betragen kann, was hinwiederum von der Art des eingesetzten Alkoxylats abhängt. Die Aufarbeitung erfolgt durch Abfiltrieren des Katalysators und anschließendes Strippen von unumgesetztem Propylen und in geringer Menge angefallener Nebenprodukte, wie oligomerem Propylen, in einem Inertgasstrom, wie Stickstoff. Die Produkte können z. B. durch ihr Molgewicht, Trübungspunkte, Säure- oder OH-Zahlen charakterisiert werden.

Die erfindungsgemäß zu verwendenden Produkte sind alkali- und säurestabile schaumarme Tenside. Sie eignen sich vor allem in technischen Reinigungsprozessen, wie der Flaschenwäsche. Sie werden wie andere übliche Tenside in Mengen von - bezogen auf Flotte - 0,01 bis 5 Gew.-%, vorzugsweise 0,1 bis 3 Gew.-%, eingesetzt.

Sie weisen außer ihrer erwähnten Säurestabilität eine gegenüber den aus der DE-AS 1 520 647 bekannten Isobutylethern verbesserte Netzwirkung auf, welche den Einsatz der obengenannten relativ geringen Mengen in den Flotten möglich macht. Auch ihre biologische Abbaubarkeit entspricht den derzeit gültigen Normen in der BRD (B ges Bl 1977, Teil I, Seite 244 ff.).

Die nun folgenden Beispiele erläutern die Erfindung. Die genannten Teile sind, soweit sie nicht ausdrücklich anders bezeichnet werden, Gewichtsteile. Hierbei erhalten sich Gewichtsteile zu Raumteilen wie kg zu l.

**Beispiel 1**

550 Teile eines 7fach ethoxylierten $C_{13}$- bis $C_{15}$-Alkohols, 450 Teile Propylen und 80 Teile eines unter dem Handelsnamen ®LEWATIT SPC 118 H bekannten sulfonierten Styrol/Divinylbenzol-Copolymerisats wurden in einem 2500 Raumteile fassenden Autoklaven 3 h bei 115°C unter Eigendruck gerührt. Nach beendeter Reaktion wurde nicht umgesetztes Propylen durch Entspannungsverdampfung aus dem System entfernt und der saure Ionenaustauscher abfiltriert sowie niedrigsiedende Anteile (130 Teile) destillativ abgetrennt. Der Rückstand (580 Teile) enthielt noch 5,5 Mol-% an Ausgangsethoxylat.

**Beispiel 2**

Durch ein mit 2400 Raumteilen an Ionenaustauscher gemäß Beispiel 1 (gequollen in 8fach ethoxyliertem $C_{13}$-bis $C_{15}$-Alkohol) gefülltes Druckrohr (Festbett) wurden bei 115°C und 30 bar kontinuierlich 150 Teile/h an 8fach ethoxyliertem $C_{13}/C_{15}$-Alkohol und 100 Teile/h Propylen geleitet. Am Reaktorausgang wurde entspannt. Der Flüssige Austrag wurde destillativ von niedrigsiedenden Anteilen befreit. Der Rückstand (165 Teile/h) enthielt noch 5,2 Mol-% an Ausgangsethoxylat.

Zum Vergleich wurden sowohl nichtendgruppenverschlossene als auch mit Butylen verschlossene Alkoholethoxylate getestet. Die Werte wurden folgendermaßen ermittelt:

Trübungspunkte: DIN 53 917
Schaum (Schlagmethode) DIN 53902
Netzvermögen: DIN 53 901 mit2 g/l Tensid bei Raumtemperatur

Die Ergebnisse der Tenside gehen aus den folgenden Tabellen hervor:

**Tabelle 1**

| Substanz | Trübungspunkt (°C) | Schaum (cm) | Netzvermögen (sec) |
|---|---|---|---|
| Beispiel 1 | 0 | 0 | 8 |
| Beispiel 1 unverschlossen | 60 | 340 | 11 |
| Beispiel 2 | 38 | 20 | 7 |
| Beispiel 2 unverschlossen | 52 | 220 | 10 |

**Tabelle 2**

| | Sofort<br><br>Schaum<br>+ 4 % $H_2SO_4$ | Nach 100 h<br>bei 65°C<br>Schaum<br>+ 4 % $H_2SO_4$ | Sofort<br><br>Schaum<br>+ 10 % $H_2SO_4$ | Nach 100 h<br>bei 65°C<br>Schaum<br>+ 10 % $H_2SO_4$ |
|---|---|---|---|---|
| $C_{13/15}$-Oxoalkohol + 7 EO + Propylen (Beispiel 1) | 0 cm | 0 cm | 0 cm | 0 cm |
| $C_{13/15}$-Oxoalkohol + 7 EO + i-Butylen | 10 cm | 50 cm | 10 cm | 70 cm |
| $C_{13/15}$-Oxoalkohol + 8 EO + Propylen (Beispiel 2) | 30 cm | 30 cm | 30 cm | 30 cm |
| $C_{13/15}$-Oxoalkohol + 8 EO + i-Butylen | 60 cm | 110 cm | 60 cm | 150 cm |

**Patentansprüche**

1. Verwendung von Verbindungen der Formel I

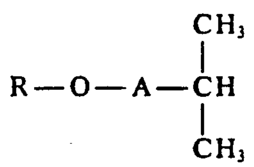

in der R für einen $C_8$- bis $C_{20}$-Alkylrest steht, und A einen Polyalkylenoxidrest mit 4 bis 30 Alkylenoxideinheiten bedeutet, der aus 100 bis 60 Mol-% Ethylenoxid- und 0 bis 40 Mol-% 1,2-Propylen- und/oder 1,2-Butylenoxidresten zusammengesetzt ist, als schaumarme, alkali- und säurestabile Tenside.

2. Verwendung von Verbindungen der Formel I nach Anspruch 1, bei denen A einen Polyethylenoxidrest bedeutet.

**Claims**

1. The use of a compound of the formula I

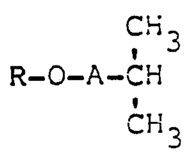

I,

where R is $C_8$-$C_{20}$-alkyl and A is a polyalkylene oxide radical containing from 4 to 30 alkylene oxide units, of which from 100 to 60 mole % are ethylene oxide units and from 0 to 40 mole % are 1,2-propylene oxide units or 1,2-butylene oxide units or a mixture of these, as a low-foam, alkali- and acid-stable surfactant.

2. The use of a compound of the formula I as Claimed in claim 1, where A is a polyethylene oxide radical.

**Revendications**

1. Utilisation, comme agents tensioactifs stables aux alcalis et aux acides et peu moussants, de composés de formule I

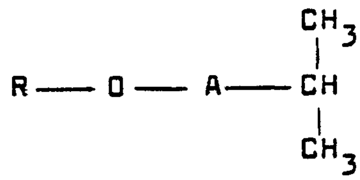

dans laquelle R représente un reste alkyle en $C_8$ à $C_{20}$ et A un reste polyalkylène-oxyde ayant 4 à 30 motifs alkylène-oxyde, qui est composé de 100 à 60 moles % de restes éthylène-oxyde et 0 à 40 moles % de restes 1,2 propylène-oxyde et/ou 1,2 butylène-oxyde.

2. Utilisation de composés de formule I selon la revendication 1 dans laquelle A représente un reste polyéthylène-oxyde.